(19) Europäisches Patentamt
European Patent Office
Office européen des brevets

(11) **EP 2 745 835 B1**

(12) **EUROPÄISCHE PATENTSCHRIFT**

(45) Veröffentlichungstag und Bekanntmachung des Hinweises auf die Patenterteilung:
**19.10.2016 Patentblatt 2016/42**

(51) Int Cl.:
*A61K 9/127* *(2006.01)*　　*A61B 5/00* *(2006.01)*
*A61K 36/82* *(2006.01)*

(21) Anmeldenummer: **13196370.4**

(22) Anmeldetag: **10.12.2013**

(54) **Neuartige Vesikel für die topische Anwendung in der Pharmazie und Kosmetik**

Novel vesicle for topical application in pharmacy and cosmetics

Nouvelles vésicules pour une utilisation topique en pharmacie et en cosmétique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2012 DE 102012025485**

(43) Veröffentlichungstag der Anmeldung:
**25.06.2014 Patentblatt 2014/26**

(73) Patentinhaber: **Blume, Gabriele**
**36396 Steinau an der Strasse (DE)**

(72) Erfinder: **Blume, Gabriele**
**36396 Steinau an der Strasse (DE)**

(74) Vertreter: **advotec.**
**Patent- und Rechtsanwälte**
**Beethovenstrasse 5**
**97080 Würzburg (DE)**

(56) Entgegenhaltungen:
**WO-A2-2011/116738**

**Beschreibung**

Die vorliegende Erfindung beruht auf einer verbesserten therapeutischen Wirksamkeit gegeben durch neuartige Lipidvesikel, die sowohl lipophile, amphiphile als auch hydrophile Wirkstoffe (kosmetisch oder pharmazeutisch relevante) beinhalten und diese in die tieferen Hautschichten zu transportieren vermögen. Die vorliegende Erfindung betrifft auch eine die Vesikel beinhaltende Zubereitung.

Diese Art der Verkapselung von hydrophilen, amphiphilen und lipophilen Wirkstoffen in Vesikeln mit einer Größe im Submikrometerbereich (nm) ist seit vielen Jahren bekannt und wird insbesondere in der Kosmetik und Pharmazie häufig eingesetzt. Vesikel, die ebenfalls die Fähigkeit besitzen, Wirkstoffe in die Haut zu transportieren sind insbesondere die Liposomen (Phosphatidylcholin / Lecithin) und Niosomen (non ionic surfactants Vesikel). Verschiedene Zusammensetzungen dieser Vesikel sind in dem Artikel "Liposomes and Niosomes as Topical Drug Delivery Systems" beschrieben (M.J. Choi & H.I. Maibach; Skin Pharmacol. Physiol. 18 (2005) 209-219). Trägersysteme, die insbesondere in der Kosmetik eingesetzt werden, führen Patravale und Mandawgade in einem Übersichtsartikel auf (Novel cosmetic delivery systems: an application update; Int. J. Cosm. Sci. 30 (2008) 19-33), sowie Mihranyan et al. (Current status and future prospects of nanotechnology in cosmetics; Progress in Materials Science 57 (2012) 875-910).

Klassische Liposomen beinhalten als membranbildende Hauptkomponente Phosphatidylcholin (PC), natürlich gewonnen aus Eiern oder Soyalecithin bzw. synthetisch hergestellt. Diese Lipide bilden spontan Vesikel (multilamellare Liposomen), wenn sie in Wasser gegeben werden. Je nach Zusammensetzung der Phospholipide (Phosphatidylcholin bestehend aus langkettigen, ungesättigten Fettsäuren) und Produktion (Hochdruckhomogenisation) können kleine einschalige und flexible Liposomen hergestellt werden. Diese Art von Liposomen ist dann befähigt, eingeschlossene Wirkstoffe in die tieferen Hautschichten zu transportieren. Artmann und andere beschreiben kleine einschalige Liposomen - hergestellt aus Soyalecithin (> 85% PC mit hohem Anteil an ungesättigten Fettsäuren, Linolsäure) - die verkapseltes hochmolekulares Heparin in die Epidermis und Dermis transportieren können (Liposomes from Soya Phospholipids as Percutaneous Drug Carriers; Arzneimittel Forschung Drug Research 40 (1990) 1365-1368). Dass der hohe Anteil an membranbildenden Phosphatidylcholin und die Flexibilität der Vesikelmembran die Voraussetzung für die Penetration der Liposomen in die Haut sind, wird deutlich im Artikel "Liposomes = Liposomes" (G. Blume; SÖFW-Journal 11 (2000) 14-17).

Eine Weiterentwicklung, um Wirkstoffe noch effizienter über die Haut in den Körper zu bringen, sind die "Transfersomen". Transfersomen unterscheiden sich zu den herkömmlichen Liposomen durch einen Anteil an randaktiven Substanzen in der Phospholipidmembran (DE 4107153). Nach topikaler Applikation dieser ultraflexiblen Trägersysteme, können die eingeschlossenen Wirkstoffe selbst aus der Dermis über den lymphatischen Weg ins Blut gelangen. So konnte der Zuckerspiegel im Blut nach Auftragen Insulin-haltiger Transfersomen gesenkt werden ("Transdermal Drug Carriers"; G. Cevc und andere; J. Contr. Release 36 (1995) 3-16).

Eine andere Verbesserung der Penetrationseigenschaften von kleinen flexiblen Liposomen wurde durch einen erhöhten Gehalt an Alkoholen in der Vesikelsuspension erzielt (US 5716638). Der hohe Anteil an Äthanol in den "Ethosomen" führt zu einer Störung / Fluidisierung der Hautlipide, die die Barriereschicht im Stratum Corneum bilden. Durch diese "Aufweichung" gelingt es den kleinen flexiblen Liposomen leichter in die tieferen Hautschichten einzudringen ("Ethosomal Carriers"; E. Touitou und andere; Drug Dev. Res. 50 (2000) 406 - 415).

Ein anderes "altes" Trägersystem sind die Niosomen, die vorwiegend aus nicht ionischen Detergentien bestehen (siehe Kumar and Rajeshwarrao "Nonionic surfactant vesicular systems for effective drug delivery - an overview; Acta Pharmaceutica Sinica B 1 (2011) 208-219). Meistens enthalten sie noch einen Membranstabilisator (Cholesterol) und einen Ladungsträger (Dicetylphosphate), der die Aggregation der Vesikel verhindern soll. Der am häufigsten eingesetzte Membranbildner ist Sorbitan Monooleate (Span 80) aber folgende Detergentien sind ebenfalls beschrieben: Sucrosemonostearate, Sucrosedistearate, POE(5) Sorbitantrioleate und Dioleylglucate. Exemplarisch werden hier einige Patente zu Niosomen aufgeführt: US-Patent 4536324 beschreibt Niosome aus PEG-10 Castoröl mit Sorbitantrioleate; US-Patent 4830857 beschreibt die Herstellung eines nicht ionischen Detergents und Niosomen aus diesem Stoff mit Cholesterol und Dicetylphosphate; US-Patent 5405615 beschreibt Niosomen aus Sucrose-distearate und einem Fettalkohol-Derivat; EP-Patent 69507488 beschreibt Niosomen aus PEG-2 bis 6-Monohexadecylether, Cholesterol und Dicetylphosphate; US-Patent 5720948 beschreibt Niosomen aus Glyceryldilaurate, Cholesterol und PEG-10-Stearylester; US-Patent 2007/0269379 beschreibt Niosomen aus N-Laurylsarcosine und Sorbitanmonolaurate (Span 20) und WO 2007/123993 beschreibt Niosomen aus Sorbitanmonostearate (Span 60), PEO-Sorbitanmonostearate (Tween 61), Cholesterol und Dicetylphosphate.

Besonders hervorzuheben ist US-Patent 5830499, das Niosomen darstellt, die Wirkstoffe besonders tief in die Haut transportieren können. Die einschaligen Vesikel bestehen aus flexiblen Membranen, die aus einem membranbildenden nicht-ionischen Detergens und einem zweiten nicht ionischen, hydrophilen Emulgator (PEG-Emulgator) gebildet werden. Hier wurde die Penetration der Vesikel selbst in den tieferen Hautschichten nachgewiesen. Explizit wird hier für den zweiten Emulgator ein HLB-Wert > 12 angeben.

Ein weiterer Wirkstoffträger für den dermalen Einsatz sind multilamellare Niosomen bestehend aus einem zweikettigen Lipid, optional aus einem einkettigen Lipid und einem Sterol. Die hier bevorzugte Zusammensetzung liegt bei 30 - 40% Glyceryldistearate, 29-37% POE-10 Stearylether und 11 -14% Cholesterol. Hier wird die Erhöhung der Wirkstoffkonzentration in der Epidermis mittels dieses Trägersystems aufgeführt.

All die hier beschriebenen und auch in der Literatur beschriebenen Niosome weisen meist Cholesterol als Stabilisator, und einen PEG-haltigen Emulgator (hydrophiler Coemulgator) auf und bestehen meist nicht aus ungesättigten Fettsäure-Estern /Ethern. Cholesterol behindert die Flexibilität der Vesikel und somit deren Penetration in die Haut.

Ein in der Kosmetik eingesetztes multilamellares Trägersystem mit optimierter Wirkstofffreigabe (Time Release Effekt) stellt das Spherulites® Trägersystem (Firma Impag) dar. Dieses Trägersystem wird in FR 2771635 beschrieben und besteht zu 30 - 50% aus Tensiden pflanzlichen Ursprungs (Zuckerester wie Sucrosepalmitat oder Sucrosed istearate).

In DE 10201003064 A1 wurden erstmalig Phospholipid-freie Vesikel beschrieben, bestehend aus einem besonders hautfreundlichen, pflanzlichen und essbaren membranbildenden Emulgator (anionisch) in Kombination mit einem weiteren einkettigen Lipid zur Stabilisierung der Vesikel. Diese Vesikel haben eine negativ geladene O-berfläche und weisen einen pH-Bereich von pH 5.5 bis pH 6.5 auf.

Daraus folgend ergab sich die neue Aufgabe, geeignete hautfreundliche Lipidzusammensetzungen zu finden, die stabile aber auch flexible Vesikel ermöglichen, die anionisch und/oder nicht ionisch sind und einen höheren pH-Bereich von pH 6.5 bis pH 9 abdecken. Die Vesikel zeichnen sich durch die Fähigkeit aus, Wirkstoffe (lipophil, amphiphil oder hydrophil) in die tieferen Hautschichten zu transportieren.

Gelöst wird die Aufgabe durch eine wässerige Zubereitung, enthaltend mindestens die folgenden Bestandteile:

- Vesikel mit einem Bilayer, gebildet aus mindestens einem membranbildenden amphiphilen Stoff der folgenden Substanzklasse: Glycerol- bzw Sorbitan-Ester / Ether von ungesättigten C18-Fettsäuren und einer hydrophilen Kopfgruppe (z.B. Polyoxyethylenglycol, Polyglycerol oder kurzkettige Karbonsäuren) sowie einem weiteren einkettigen ungesättigtem C18 bis C24 Fettsäurederivat zur Membranstabilisierung (HLB-Wert< 10);
- ein wässeriges Medium, das sowohl im Vesikelinneren als auch im Trägervehikel dieselbe Zusammensetzung aufweist;
- mindestens einen und / oder mehrere kosmetisch und / oder pharmazeutisch relevante Wirkstoffe, die hydrophil, amphiphil oder lipophil sein können, wobei die Vesikel einen pH-Wert im Bereich von 7 bis 9 aufweise.

Die Aufgabe wird ferner gelöst durch eine Zubereitung enthaltend die Vesikel wie sie im Folgenden beschrieben werden.

Die erfindungsgemäßen einschaligen Vesikel haben vorzugsweise eine Größe von 80 - 250 nm mit einer sehr geringen Polydispersität (< 0.2).

Sie liegen dispergiert in einer wässerigen Lösung vor.

In einer bevorzugten Ausführungsform enthält die erfindungsgemäße Zubereitung noch einen Stabilisator (z.B. wasserlöslichen Alkohol oder Polyol) und ist damit als konservierungsmittelfrei anzusehen.

Die Vesikel enthaltende wässerige Zubereitung kann selbst als Kosmetikum / Pharmazeutikum eingesetzt werden, je nach enthaltenem Wirkstoff. Darüber hinaus lässt die Vesikel enthaltende wässerige Zubereitung sich in die Wasserphase einer weiteren Zubereitung wie eine Emulsion, Lotion, Creme oder unterschiedliche Formen von Gelen einarbeiten oder kann selbst verdünnt mit einem zugesetzten Verdicker als Sprühformulierung angewendet werden.

Die vorliegende Erfindung weist eine Verkapselungseffizienz auf, die sich zu der normalen liposomalen Verkapselung dadurch auszeichnet, dass Wirkstoffe wie z.B. Polyphenole (Phenolsäuren und Flavonoide) in höherer Konzentration stabil im Vesikel eingeschlossen werden können.

Weitere Vorteile der erfindungsgemäßen Zubereitung sind die hohe Stabilität der Komposition auch in emulgator-haltigen kosmetischen und pharmazeutischen Formulierungen, ein gutes Hautgefühl und schnelles Eindringen in die Haut. Erfindungsgemäß verwendbare amphiphile membranbildende Substanzen sind ausgewählt aus der Gruppe von Glycerol-Estern / Ether bzw Sorbitan-Estern / Ether von einfach oder mehrfach ungesättigten C18-Fettsäuren wie z.B.: Ölsäure, Linolsäure, Linolensäure oder Ricinolsäure und einer hydrophilen Kopfgruppe (z.B. Polyethylenglycol, Polyglycerol und kurzkettige Karbonsäuren). Erfindungsgemäß besonders bevorzugte Substanzen sind Polyglyceryl-3 Dioleate (Plurol Oleique); Polyglyceryl-3 Polyricinoleate (Crester PR ON Syn Gly) und Polyglyceryl-5 Dioleate (Dermofeel G5DO) sowie Glyceryl Citrate/Lactate/Linoleate/Oleate (Imwitor 375) bzw. Sorbitan-dioleate (Undessa) undSorbitansesquioleate (Crill 43). Der hohe Anteil an ungesättigten und mehrfach ungesättigten Fettsäuren ermöglicht die Bildung von Vesikeln mit einer flexiblen Membran. Die Flexibilität ist eine Voraussetzung für die Penetration der Vesikel in die Haut nach topischer Applikation. Diese amphiphilen Substanzen mit langen Alkylketten zeichnen sich durch eine hohe Hautverträglichkeit aus und können kosmetische und pharmazeutische Effekte erzielen (z. B: Erhöhung der Hautglätte, Reduzierung von Metalloproteasen, Reduzierung von Hautunreinheiten).

Die Menge an membranbildenden Emulgator in den erfindungsgemäßen Zubereitungen beträgt vorzugsweise zwischen 5 und 15 Gew.-% besonders bevorzugt zwischen 8 und 12 Gew.-%.

Neben der membranbildenden amphiphilen Substanz wird ein lipophiler einkettiger Stabilisator eingesetzt. Die Stabilisatoren sind ausgewählt aus der Gruppe der Derivate von langkettigen (C18 bis C 24) ungesättigten Fettsäuren z.B.: Sorbitan Monooleate (Span 80), Diglyceryl-Monooleate (Nikkol DGMO); Polyglyceryl$_x$-Monooleate (Hydrior Produkte, Cremer Care), Polyethylenglycol$_x$-Monooleate (Brij-Typen), Polyethylensorbitan-Monooleate (Tween-Typen), Sucrose Monooleate (Surfhope C1715), Ethyl Oleate (Crodamol OE), Ethyl Linoleate und Ethyl Eicosapentanoate und ungesättigte C18-C24 Alkohole.

Dieser Stabilisator ist nötig, um höhere Einschlüsse von Wirkstoffen (z.B. Polyphenolen) zu erzielen, die Penetrationseigenschaften der Vesikel zu verbessern und die Vesikelstabilität in kosmetischen und pharmazeutischen Formulierungen zu erhöhen.

Diese stabilisierenden Zusatzstoffe mit langer Alkylkette zeichnen sich durch eine hohe Hautverträglichkeit aus und können kosmetische und pharmazeutische Effekte erzielen (z. B: Erhöhung der Hautglätte, Reduzierung von Metalloproteasen, Reduzierung von Hautunreinheiten, Erhöhung der Hautfeuchtigkeit, entzündungshemmende Eigenschaften).

Besonders bevorzugt wird Ethyl Oleate (EO) oder kurzkettige ethoxylierte Fettalkohole (PEG-2 Monooleate) (HLB-Werte < 5) zur Stabilisierung der Vesikelmembran eingesetzt.

Die Menge an membranbildenden Emulgator in den erfindungsgemäßen Zubereitungen beträgt zwischen 5 und 15 Gew.-%. und die des membran-stabilisierenden einkettigen Lipids zwischen 15 und 5 Gew.-%. Beide Komponenten bilden die Schale der Vesikel.

Die erfindungsgemäße Zubereitung kann zusätzlich noch einen und / oder mehrere lipophile Wirkstoffe in der Vesikelmembran enthalten. Als solche Substanzen sind alle Stoffe anzusehen, die sich in einem lipophilen Medium bei Raumtemperatur oder unter Wärme lösen lassen und kosmetisch und/oder pharmazeutisch relevante Effekte in oder auf der Haut ausüben.

Ein Auszug der kosmetisch und / oder dermatologisch wirksamen lipophilen Substanzen, die eingesetzt werden können - bevorzugt pflanzlichen Ursprungs - sind: Anti-Cellulite wirksame Substanzen (z.B: CLA); Anti-Elastase und Anti-Collagenase wirksame Stoffe (z.B: ungesättigte Fettsäuren wie Ölsäure oder EPA); antiinflammatorisch wirksame Stoffe (z.B: EPA = Eicosapentaensäure); Antioxidantien (z.B: Vitamin C-Palmitate und Tocopherol; α-Liponsäure), Ceramide (z.B: verschiedene Ceramide der Firma Cosmoferm); hautberuhigende und hautglättende Wirkstoffe (z.B: Bisabolol); Moisturiser (z.B: Glycerol Monoisostearate, Sucrose Polysoyate); Phytosterole (wie β-Sitosterol aus Maisfaseröl); Radikalfänger (z.B: Ubichinol-Derivate wie Coenzym Q10); Saponine (z.B: vom Ginseng, Süßholzwurzel und Rosskastanie); sebumreduzierende Substanzen (z.B: 10-Hydroxydecansäure); Stoffe, die die Durchblutung und damit die Versorgung der Haut fördern (z.B: lipophile Nikotinsäureester); Terpene (Ursolsäure, Rosmarinsäure, Betulinsäure, Boswelliasäure und Bryonolsäure; kosmetisch und dermatologisch relevante Terpene sind aufgeführt in der Pharmazeutischen Zeitung Heft 22; 2006 von Sebastian Jäger u.a.); Vitamine (Retinol und Derivate, Vitamin E und Derivate wie Tocotrienole oder Carotine und Carotinoide wie Lycopene, Lutein oder Fucoxanthin, Vitamin D und Derivate) und lipophile Peptide als Anti-Ageing Produkt (z.B. Ester von 2-10 Aminosäuren, die an Palmitinsäure gebunden sind)

Alle diese Wirkstoffe sollen die Hautalterung und / oder das Photoageing (durch UV- bzw. Umweltbelastung) verhindern bzw. inhibieren; die Synthese von dermalen und epidermalen Makromolekülen stimulieren bzw. deren Degradation verhindern; die Proliferation von Fibroblasten und Keratinozyten anregen und somit den Schutz und die Erhaltung einer gesunden Haut bewirken.

Pharmazeutisch eingesetzte erfindungsgemäße Zubereitungen enthalten eine sichere und wirksame Menge eines pharmazeutisch wirksamen Stoffes, welches unter den Arzneimitteln gegen Akne (z. B. Isotretinoin und Isolutrol), nichtsteroidalen entzündungshemmenden Mitteln (z.B. Diclofenac), Mitteln zur Heilung von Wunden, Mitteln zur Behandlung von Hauterkrankungen / Ekzeme (z.B. Corticosteroide), Lokalanästhetika (z.B. Lidocain), Mittel gegen Pilzinfektionen (z.B. Terbinafin), antimikrobielle Arzneimittel, Mittel gegen Psoriasis (z.B. Calcipotriol), Mittel zur Stimulierung des Haarwuches (z.B. Minoxidil), Mittel gegen chronische venöse Insuffizienz, Mittel gegen Herpesinfektionen (z.B. Aciclovir), Antihistaminika (z.B. Dimetinden), Hormone (z.B. Östrogene, Gestagene oder Androgene) und Mitteln gegen Hautkrebs (z.B.Diclofenac und Psoralene) zu finden sein können.

Alle diese relevanten pharmazeutische Wirkstoffe sollen mittels des Trägersystems effektiv in die tieferen Hautschichten transportiert werden, was zu einer höheren Bioverfügbarkeit am Wirkort führt. Damit kann zum Teil die Wirkstoffkonzentration gesenkt werden und eventuelle Nebenwirkungen vermieden werden. Ferner kann die Verkapselung der Wirkstoffe, diese stabilisieren und auch Nebenwirkungen wie Hautirritationen (z.B. bei Vitamin A-Derivaten) verhindern.

In einer erfindungsgemäß bevorzugten Ausführungsform liegt das Gewichtsverhältnis von lipophilen Bestandteilen in der Membran bei membranbildendem Emulgator zu Stabilisator und / oder Wirkstoff im Bereich von 1:1 bis 4:1.

Die erfindungsgemäße Zubereitung enthält einen wässerigen Innenkern, in dem ein oder mehrere geeignete Wirkstoffe gelöst sein können sowie im Trägervehikel.

Kosmetisch und pharmazeutisch relevante Wirkstoffe können u.a. aus folgenden Stoffgruppen ausgewählt sein:

Aminosäuren, Peptide, Proteine und Enzyme:

Weiterhin können die erfindungsgemäßen Zusammensetzungen Monomere, Oligomere und Polymere von Aminosäuren sowie Ester und/oder physiologisch verträgliche Metallsalze dieser Substanzen enthalten.

Die Monomere der Aminosäuren können u.a. sein: sind ausgewählt aus Alanin, Arginin, Asparagin, Asparaginsäure, Canavanin, Citrullin, Cystein, Glutamin, Glycin, Histidin, Hydroxylysin, Hydroxyprolin, Leucin, Lysin, Methionin, Prolin, Serin, Threonin, Tyrosin und Valin.

Die Oligomere der Aminosäuren sind bevorzugt ausgewählt aus Di-, Tri-, Tetra-, Penta-, Hexa-oder Pentadecapeptiden, die acyliert und/oder verestert sein können. Zahlreiche dieser Aminosäureoligomere stimulieren die Collagensynthese und werden bevorzugt als Wirkstoffe gegen die Hautalterung verwendet.

Die Polymere der Aminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Pflanzliche Proteinhydrolysate können sein: z. B. Soja-, Weizen-, Mandel-, Erbsen-, Kartoffel- und Reisproteinhydrolysate.

In einer weiteren Ausführungsform können Enzyme verkapselt sein, ausgewählt u.a. aus DNA-Reparaturenzymen (z.B. die Photolyase); anti-oxidativ wirkende Enzyme (u.a. Superoxydismutase), Proteasen (u.a. Trypsin) und Lipasen.

Wässerige und/ oder alkoholische Extrakte aus Pflanzen, Pilzen oder Algen:

Der Pflanzenextrakt kann beispielsweise durch Extraktion der gesamten Pflanze, a-ber auch ausschließlich durch Extraktion aus Blüten und/oder Blättern und/oder Samen und/oder anderen Pflanzenteilen, hergestellt werden. Die verwendeten Algenextrakte stammen u.a. aus Grünalgen, Braunalgen, Rotalgen oder Blaualgen (Cyanobakterien). Die zur Extraktion eingesetzten Algen und Pilze (speziell Hefen) können sowohl natürlichen Ursprungs sein als auch durch biotechnologische Prozesse gewonnen werden und gewünschtenfalls gegenüber der natürlichen Form verändert sein. Als Extraktionsmittel zur Herstellung der Pflanzenextrakte können beispielsweise Wasser, Alkohole sowie deren Mischungen verwendet werden. Die Wasserdampfdestillation fällt erfindungsgemäß unter die bevorzugten Extraktionsverfahren.

Oligonukleotide:

Die erfindungsgemäßen Zusammensetzungen können Oligonukleotide enthalten. Mononucleotide sind u.a. Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).

Ein erfindungsgemäß besonders bevorzugtes Oligonucleotid ist das Thymidin-Dinucleotid (pTT), das DNA-reparierende Eigenschaften als auch bräunend wirkt.

Zuckerbestandteile:

Weiterhin können die erfindungsgemäßen Zusammensetzungen ein Mono-, Oligo- oder Polysaccharid oder deren Derivate enthalten.

Geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin.

Erfindungsgemäß geeignete Polysaccharide sind aus mehr als zehn Monosaccharideinheiten zusammengesetzt. Besonders bevorzugt eingesetzt werden können u.a. die Mucopolysaccharide (Hyaluronsäure und ihre Derivate, Chondroitin und seine Derivate, Heparin und seine Derivate; Fucoidan und seine Derivate, Keratan und seine Derivate) und die β-Glucane.

Ein besonders bevorzugter Komplex verschiedener Zuckervarianten ist das Tonsolin (von Gova Group), das Mannose, Glucose, b-Glucan und Hyaluronsäure enthält und eine schnelle und sichere Bräunung der Haut erzielt.

Hydroxy-und Ketocarbonsäuren und deren Salze:

Weitere bevorzugte hydrophile Wirkstoffe sind die $\alpha$-Hydroxycarbonsäure, $\alpha$-Ketocarbonsäure oder ß-Hydroxycarbonsäure oder deren Ester-, Lacton-oder Salzform. Dazu gehören u.a. Glycolsäure, Milchsäure, Weinsäure, Citronensäure, 2-Hydroxyhexadecansäure, Mandelsäure, Äpfelsäure, Glucarsäure, Galactarsäure, Mannarsäure, Gluconsäure, Brenztraubensäure, Salicylsäure, Glucuronsäure und Galacturonsäure.

Vitamine:

In einer weiteren Ausführungsform können die die erfindungsgemäßen Zusammensetzungen ein wasserlösliches Vitamin enthalten aus den Vitamingruppen B und C, und den Estern der vorgenannten Substanzen.

Zur Vitamin B-Gruppe oder zu dem Vitamin B-Komplex gehören unter anderem - Vitamin $B_1$, (Thiamin), Thiaminhydrochlorid , Vitamin $B_2$ (Riboflavin), Riboflavin oder seine Derivate, Vitamin $B_3$ (Nicotinsäure und Nicotinsäureamid),Vitamin $B_6$- Gruppe (Pyridoxin, Pyridoxamin und Pyridoxal) und Vitamin $B_7$ (Biotin).

Vitamin C (Ascorbinsäure) wird bevorzugt als folgende stabile Derivate eingesetzt: Ascorbylacetat, Mg-Ascorbylphosphat, Na-Ascorbylphosphat, Natrium-und Magnesiumascorbat, Dinatrium-ascorbylphosphat und -sulfat, Kaliumascorbyltocopherylphosphat und Ascorbylglucosid.

Polyphenole:

Polyphenole sind aromatische Verbindungen, die zwei oder mehr direkt an den aromatischen Ring gebundene Hydroxylgruppen enthalten und zu den sekundären Pflanzenstoffen gerechnet werden. Natürliche Polyphenole kommen in Pflanzen als bioaktive Substanzen wie Farbstoffe (Flavonoide, Anthocyane), Geschmacksstoffe und Tannine vor. Zu den Polyphenolen gehören die Phenolsäuren und die Flavonoide, die aufgrund ihrer kosmetischen und/oder pharmazeutischen Wirkung (u.a. antioxidative, entzündungshemmende antibakterielle und antivirale Eigenschaften) in die Vesikel verkapselt werden können:

Zu den erfindungsgemäß einsetzbaren Phenolsäuren gehören u.a. Gallussäure, Vanillinsäure, Usninsäure, Ferulasäure, Ellagsäure und Kaffesäure

Zu den erfindungsgemäßein setzbbaren Flavonoiden und/oder deren glycosidische Derivate gehören: u.a. Flavone (Luteolin, Apigenin), Flavonole (Baicalin, Quercetin, Rutin, Kaempferol, Myricetin), Flavanole (Catechin, Gallocatechin, Epicatechin, Epigallocatechingallat, Resveratol, Silymarin, Theaflavin), Flavanone (Aspalathin, Hesperetin, Naringenin), Flavanonole (Taxifolin), Isoflavone (Genistein, Daidzein, Licoricidin) und die Anthocyanidine oder Anthocyane (natürliche Farbstoffe).

Bräunung:

Erfindungsgemäß können in die Vesikel folgende Wirkstoffe zu Induzierung der Pigmentierung eingesetzt werden: Erythrulose; Unipertan Veg (Gemisch aus Acetylthy-rosin, Riboflavin und hydrolysiertem pflanzenlichen Protein von Induchem); Tonsolin (Zuckermischung von Gova) das Dinucleotid pTT und das melaninstimulierende Peptid (a-Melanotropin).

Hautaufheller:

In einer weiteren bevorzugten Ausführungsform kann die erfindungsgemäßen Zusammensetzung einen hautaufhellenden Wirkstoff, der Ascorbinsäure und deren Ester sein kann (Natriumascorbylphosphat und Magnesiumascorbylphosphat) oder das Hydroxytyrosol (Cayoma Olive von Qenax).

Antioxidatien:

Die erfindungsgemäßen Zusammensetzungen können ferner Antioxidantien enthalten, zum Beispiel Grüntee Extrakt (Teavigo), Imidazole (z. B. Urocaninsäure) und deren Derivate, Peptide (u.a. D,L-Carnosin, D-Carnosin, L-Carnosin); Säuren und deren Derivate(Chlorogensäure, Huminsäure, Gallensäure, Rutinsäure und Ferulasäure) Vitamine (Vitamin C und seine Derivate wie Magnesiumascobylphosphate); Enzyme (Katalase und Superoxid-Dismutase) Zink und dessen Derivate (z. B. ZnO, $ZnSO_4$), Selen und dessen Derivate (z. B. Selen-Methionin), Stilbene und deren Derivate (z. B. Resveratol)

Alle diese Wirkstoffe sollen die Hautalterung und / oder das Photoageing (durch UV- bzw. Umweltbelastung) verhindern bzw. inhibieren; die Synthese von dermalen und epidermalen Makromolekülen stimulieren bzw. deren Degradation verhindern; die Proliferation von Fibroblasten und Keratinozyten anregen und somit den Schutz und die Erhaltung einer gesunden Haut bewirken.

In einer erfindungsgemäß bevorzugten Ausführungsform liegt das Gewichtsverhältnis der lipophilen Bestandteile der Membran bei 5-15 Gew.% (Amphiphile Substanz : Stabilisator: Wirkstoff) besonders bevorzugt zwischen 8 und 12 Gew.% und die des eingesetzten hydrophilen Wirkstoffes zwischen 0,1 bis 20 Gew.% bezogen auf die Gesamtzubereitung. Die Einsatzkonzentration des hydrophilen Wirkstoffes kann variieren und ist stark abhängig vom jeweiligen Lösungsverhalten des Stoffes.

Die erfindungsgemäßen Zubereitungen können weiterhin wenigstens einen Stabilisator, der vorzugsweise ein wasserlöslicher Alkohol ist, enthalten. Der Stabilisator / Solvent wird vorzugsweise in einer Konzentration von 10 - 20 Gew.-%, bezogen auf die gesamte Zubereitung, eingesetzt. Geeignet sind je nach Darreichungsform einwertige Alkohole wie z. B. Ethanol, Propanol, Isopropanol oder natürlicher Phenylethylalkohol. Weiterhin geeignet sind wasserlösliche Polyole. Hierzu zählen wasserlösliche Diole, Triole und höherwertige Alkohole. Unter den Diolen eignen sich $C_2$ bis $C_{10}$-Diole insbesondere 1,2-Propylenglycol, 1,2-Butylenglycol, 1,3-Butylenglycol und 1,4-Butylenglycol, 1,2-Pentadiol, 1,6-Hexandiol und 1,2 Octandiol. Weiterhin bevorzugt geeignet sind Glycerin und insbesondere Diglycerin und Triglycerin, 1,2,6-Hexantriol sowie die Dipropylenglycole. Für die Herstellung der erfindungsgemäßen Vesikel werden bevorzugt Ethanol, Phenylethylalkohol oder 1,2 Pentandiol (Hydrolite 5 von Symrise) eingesetzt, in Konzentrationen von 5 bis 30 Gew.-% besonders bevorzugt von 5 - 15 Gew.-% bezogen auf die gesamte Formulierung, sodass somit auf eine andere Konservierung (klassische Konservierungsmittel) verzichtet werden kann. Die erfindungsgemäße Zubereitung kann deshalb als konservierungsmittelfrei deklariert werden.

Die hier beschriebene Erfindung beinhaltet ebenfalls den Gebrauch der Vesikel kosmetisch, dermatologisch und pharmazeutisch interessanten wässerigen Formulierungen (Sprühformulierung, unterschiedlichste Gele, Lotionen und Cremes) sowie deren Anwendung zur Behandlung, zum Schutz und Pflege der Haut, Haare, Nägel und Lippen.

Im Folgenden soll das Verfahren zur Herstellung einer erfindungsgemäßen Zubereitung anhand von einigen Ausführungsbeispielen und Abbildungen, die nicht als einschränkend zu verstehen sind, demonstriert werden.

Beispiel 1

Verkapselung des hydrophilen Wirkstoffes "Grüntee" (Teavigo von DSM)

Liposomen:

10 g Phopsholipid (Phospholipon 85 G von Lipoid) wird in 16 g Äthanol gelöst Diese Lösung wurde unter Homogenisieren zu 74 g wässeriger Grünteelösung gegeben. Anschließend wurde alles für 2 Minuten bei 200 bar hochdruckhomogenisiert. Teilchengröße: 225 nm $\pm$ 0,356 (extrem inhomogene Verteilung); pH = 6.58

Neue Vesikel:

7 g Sorbitan-Dioleate + 3 g Crodamol OE wurden in 16 g Äthanol gelöst Diese Lösung wurde unter Homogenisieren zu 74 g wässeriger Grünteelösung gegeben. Anschließend wurde alles für 2 Minuten bei 200 bar hochdruckhomogenisiert. Teilchengröße: 157 nm $\pm$ 0,058 (sehr homogene Verteilung); pH = 9,01

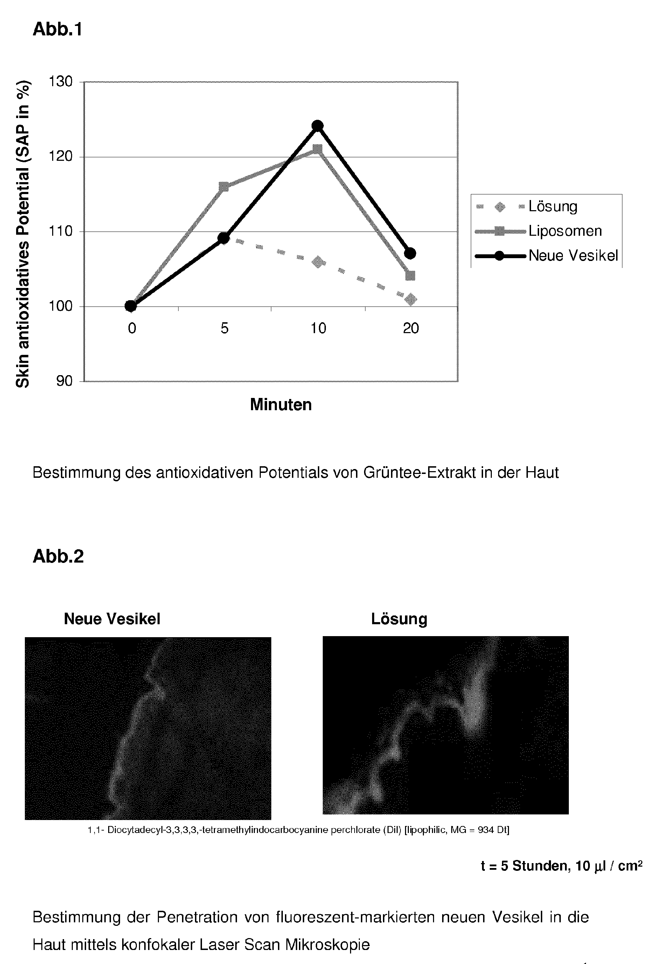

Eine Messung der Penetration von Grüntee- Extrakt mittels der 2 verschiedenen Trägersysteme in die Haut wurde mit Hilfe der ESR-Spektroskopie bestimmt. Die Haut hat einen natürlichen anti-oxidativen Schutz, gegeben durch hauteigene enzymatische und nicht-enzymatische Wirksubstanzen. Dieses anti-oxidative Potential der Haut (Skin Antioxidative Potential, SAP) kann gemessen werden, in dem die Epidermis mit Testradikalen inkubiert wird und Reduktion der Radikale mittels ESR verfolgt wird (entspricht 100%). Topisch applizierte Antioxidatien, die in die Haut penetrieren können, können dann das SAP erhöhen. Die neuen Vesikel zeigen vergleichbare Penetrationseigenschaften wie die herkömmlichen flexiblen Liposomen (Abb.1).

Beispiel 2

Verkapselung des lipophilen roten Fluoreszenzfarbstoffes Dil

$$DiI = 1,1\text{-}\ Diocytadecyl\text{-}3,3,3,3,\text{-}tetramethylindocarbocyanine\ perchlorate\ \ [lipophilic,\ MG = 934\ Dt]$$

Neue Vesikel:

8 g Cresta PR ON Syn Gly + 2 g Codamol OE + 5 mg Dil wurden in 16 g Äthanol gelöst.

Diese Lösung wurde unter Homogenisieren zu 73,995 g Wasser gegeben. Anschließend wurde alles für 2 Minuten bei 200 bar hochdruckhomogenisiert.

Teilchengröße: 225 nm $\pm$ 0,163 (homogene Verteilung); pH = 8.13

Die Penetration in die Haut wurde ex vivo mit der konfokalen Laser Scan Mikroskopie ermittelt. Deutlich zeigt sich, dass der verkapselte lipophile Wirkstoff mittels der Vesikel in die tieferen Hautschichten gebracht wird. Dagegen wird das Dil im Stratum corneum zurückgehalten, wenn er als wässerige äthanolischer Lösung aufgetragen wird (Abb. 2).

Beispiel 3

Herstellung von Vesikeln ohne Hochdruckhomogenisation

Neue Vesikel:

6 g Tween 80 + 4 g Brij 02 wurden in 16 g Äthanol gelöst.

Diese Lösung wurde unter Homogenisieren zu 74 g PBS (pH 7.0) gegeben. Anschließend wurde alles für 5 Minuten bei 15.000 U/min nachhomogenisert (UltraThurax).

Teilchengröße: 117 nm $\pm$ 0,210 (homogene Verteilung); pH = 7.02

**Patentansprüche**

1.   Wässerige Zubereitung, enthaltend mindestens die folgenden Bestandteile:

- Vesikel mit einem Bilayer, gebildet aus mindestens einem membranbildenden amphiphilen Stoff der folgenden Substanzklasse: Glycerol- bzw Sorbitan-Ester / Ether von ungesättigten C18-Fettsäuren und einer hydrophilen Kopfgruppe (z.B. Polyoxyethylenglycol, Polyglycerol oder kurzkettige Karbonsäuren) sowie einem weiteren einkettigen ungesättigtem C18 bis C24 Fettsäurederivat zur Membranstabilisierung (HLB-Wert < 10);
- ein wässeriges Medium, das sowohl im Vesikelinneren als auch im Trägervehikel dieselbe Zusammensetzung aufweist;
- mindestens einen und / oder mehrere kosmetisch und / oder pharmazeutisch relevante Wirkstoffe, die hydrophil, amphiphil oder lipophil sein können,

wobei die Vesikel einen pH-Wert im Bereich von 7 bis 9 aufweisen.

2.   Zubereitung nach Anspruch 1, **dadurch gekennzeichnet, dass** die Vesikel eine Größe von 80 bis 250 nm besitzen bei einer sehr homogenen Größenverteilung.

3.   Zubereitung nach einem der Ansprüche 1 bis 2, **dadurch gekennzeichnet, dass** es sich bei der amphiphilen Substanz bevorzugt um Dioleat-Ester / Ether handelt.

4.   Zubereitung nach einem der Ansprüche 1 bis 3, **dadurch gekennzeichnet, dass** es sich bei dem membran-stabilisierenden einkettigen Lipid bevorzugt um einen Fettsäureester/ether mit einen HLB-Wert < 5 handelt.

5.   Zubereitung nach einem der Ansprüche 1 bis 4, **dadurch gekennzeichnet, dass** die Vesikelmembran einen kosmetisch und/oder pharmazeutisch relevante lipophilen Wirkstoff enthalten kann.

6.   Zubereitung nach einem der Ansprüche 1 bis 5, **dadurch gekennzeichnet, dass** das Gewichtsverhältnis von amphiphiler Substanz zu Membranstabilisator + lipophilen Wirkstoff bei 1:1 bis 4:1 liegt.

7.   Zubereitung nach einem der Ansprüche 1 bis 6, **dadurch gekennzeichnet, dass** das wässerige Medium im Vesikelinneren und im Trägervehikel kosmetisch und pharmazeutisch relevante hydrophile Wirkstoffe enthalten kann.

8.   Zubereitung nach Anspruch 7, **dadurch gekennzeichnet, dass** sie weiterhin mindestens einen hydrophilen Stabilisator enthält.

9.   Zubereitung nach einem der Ansprüche 1 bis 8 zur therapeutischen Behandlung des menschlichen oder tierischen Körpers.

10.   Verwendung der Zubereitung nach einem der Ansprüche 1 bis 8 für einen kosmetischen und/oder nicht- therapeutischen, dermatologischen Zweck.

11.   Vesikel mit einem Bilayer, gebildet aus mindestens einem membranbildenden amphiphilen Stoff der folgenden Substanzklasse: Glycerol- bzw Sorbitan-Ester / Ether von ungesättigten C18-Fettsäuren und einer hydrophilen Kopfgruppe (z.B. Polyoxyethylenglycol, Polyglycerol oder kurzkettige Karbonsäuren) sowie einem weiteren einkettigen ungesättigtem C18 bis C24 Fettsäurederivat zur Membranstabilisierung (HLB-Wert < 10),
wobei die Vesikel einen pH-Wert im Bereich von 7 bis 9 aufweisen.

**Claims**

1. An aqueous preparation, comprising at least the following components:

   - vesicles comprising a bilayer formed out of at least one membrane-forming amphiphilic substance of the following class of compounds:

     glycerol ester/ether or sorbitan ester/ether of unsaturated C18 fatty acids and a hydrophilic head group (e.g. polyoxyethylene glycol, polyglycerol or short-chained carboxylic acids) as well as another single-chained unsaturated C18 to C24 fatty acid derivative for membrane stabilisation (HLB value < 10);

     - an aqueous medium which exhibits the same composition in the vesicle interior as well as in the dispersion vehicle;
     - at least one and/or more cosmetically and/or pharmaceutically relevant active ingredients which can be hydrophilic, amphiphilic or lipophilic,

   wherein the vesicles have a pH value in the range of 7 to 9.

2. The preparation according to claim 1, **characterised in that** the vesicles have a size of 80 to 250 nm at a very homogeneous size distribution.

3. The preparation according to any one of the claims 1 or 2, **characterised in that** the amphiphilic substance is preferably a dioleate ester/ether.

4. The preparation according to any one of the claims 1 to 3, **characterised in that** the membrane-stabilising single-chain lipid is preferably a fatty acid ester/ether having an HLB value < 5.

5. The preparation according to any of the claims 1 to 4, **characterised in that** the vesicle membrane can comprise a cosmetically and/or pharmaceutically relevant lipophilic active ingredient.

6. The preparation according to any one of the claims 1 to 5, **characterised in that** the weight ratio of the amphiphilic substance to the membrane stabiliser + lipophilic active ingredient is 1:1 to 4:1.

7. The preparation according to any one of the claims 1 to 6, **characterised in that** the aqueous medium in the vesicle interior and in the dispersion vesicle can comprise cosmetically and pharmaceutically relevant hydrophilic active ingredients.

8. The preparation according to claim 7, **characterised in that** the preparation further comprises at least one hydrophilic stabiliser.

9. The preparation according to any one of the claims 1 to 8 for the therapeutic treatment of human or animal bodies.

10. Use of the preparation according to any one of the claims 1 to 8 for a cosmetic and/or non-therapeutic, dermatological purpose.

11. Vesicles comprising a bilayer formed out of at least one membrane-forming amphiphilic substance of the following class of compounds:

    glycerol ester/ether or sorbitan ester/ether of unsaturated C18 fatty acids and a hydrophilic head group (e.g. polyoxyethylene glycol,
    polyglycerol or short-chained carboxylic acids) as well as another single-chained unsaturated C18 to C24 fatty acid derivative for membrane stabilisation (HLB value < 10),
    wherein the vesicles have a pH value in the range of 7 to 9.

**Revendications**

1. Préparation aqueuse, comprenant au moins les composants suivants :

   - des vésicules ayant une bicouche formée d'au moins une substance amphiphile formant membrane de la classe de substance suivante:

     glycérol ester/éther ou sorbitane esther/éther de C18 acides gras insaturés et un groupe de tête hydrophile (par exemple :

        polyoxyéthylène glycérol, polyglycérol ou des acides carboxyliques à chaîne courte) aussi bien qu'un autre C18 à C24 dérivé d'acides gras insaturés à chaîne unique pour la stabilisation de la membrane (valeur HLB < 10) ;

   - un médium aqueux qui a la même composition tant à l'intérieur de la vésicule que dans le véhicule porteur ;
   - au moins une et/ou plusieurs ingrédients actifs pertinents du point de vue cosmétique ou pharmaceutique, les ingrédients actifs étant hydrophiles, amphiphiles ou lipophiles,

   dans laquelle les vésicules ont une valeur pH de 7 à 9.

2. Préparation selon la revendication 1, **caractérisée en ce que** les vésicules ont une taille de 80 à 250 nm à une distribution granulométrique très homogène.

3. Préparation selon l'une quelconque des revendications 1 à 2, **caractérisée en ce que,** de préférence, la substance amphiphile est un dioléate ester/ether.

4. Préparation selon l'une quelconque des revendications 1 à 3, **caractérisée en ce que,** de préférence, le lipide à chaîne unique stabilisateur de la membrane est un ester/éther d'acides gras insaturés ayant une valeur HLB < 5.

5. Préparation selon l'une quelconque des revendications 1 à 4, **caractérisée en ce que** la membrane de la vésicule peut comprendre un ingrédient actif lipophile pertinent du point de vue cosmétique et/ou pharmaceutique.

6. Préparation selon l'une quelconque des revendications 1 à 5, **caractérisée en ce que** le rapport pondéral de la substance amphiphile au stabilisateur de la membrane + l'ingrédient actif lipophile est de 1:1 à 4:1.

7. Préparation selon l'une quelconque des revendications 1 à 6, **caractérisée en ce que** le médium aqueux à l'intérieur de la vésicule et dans le véhicule porteur peut comprendre des ingrédients actifs hydrophiles pertinents du point de vue cosmétique et pharmaceutique.

8. Préparation selon la revendication 7, **caractérisée en ce que** la préparation a en outre au moins un stabilisateur de membrane.

9. Préparation selon l'une quelconque des revendications 1 à 8 pour le traitement thérapeutique du corps humain ou animal.

10. Usage de la préparation selon l'une quelconque des revendications 1 à 8 pour un but dermatologique cosmétique et/ou non thérapeutique.

11. Vésicules ayant une bicouche formée d'au moins une substance amphiphile formant membrane de la classe de substance suivante:

    glycérol ester/éther ou sorbitane esther/éther de C18 acides gras insaturés et un groupe de tête hydrophile (par exemple :

       polyoxyéthylène glycérol, polyglycérol ou des acides carboxyliques à chaîne courte) aussi bien qu'un autre C18 à C24 dérivé d'acides gras insaturés à chaîne unique pour la stabilisation de la membrane (valeur HLB < 10) ;
       dans lesquelles les vésicules ont une valeur pH de 7 à 9.

EP 2 745 835 B1

## Abb.1

Bestimmung des antioxidativen Potentials von Grüntee-Extrakt in der Haut

## Abb.2

**Neue Vesikel**   **Lösung**

1,1- Diocytadecyl-3,3,3,3,-tetramethylindocarbocyanine perchlorate (DiI) [lipophilic, MG = 934 Dt]

**t = 5 Stunden, 10 µl / cm²**

Bestimmung der Penetration von fluoreszent-markierten neuen Vesikel in die Haut mittels konfokaler Laser Scan Mikroskopie

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4107153
- US 5716638 A
- US 4536324 A
- US 4830857 A
- US 5405615 A
- EP 69507488 A
- US 5720948 A
- US 20070269379 A
- WO 2007123993 A
- US 5830499 A
- FR 2771635
- DE 10201003064 A1

**In der Beschreibung aufgeführte Nicht-Patentliteratur**

- **M.J. CHOI ; H.I. MAIBACH.** *Skin Pharmacol. Physiol.,* 2005, vol. 18, 209-219
- Novel cosmetic delivery systems: an application update. *Int. J. Cosm. Sci.,* 2008, vol. 30, 19-33
- **MIHRANYAN et al.** Current status and future prospects of nanotechnology in cosmetics. *Progress in Materials Science,* 2012, vol. 57, 875-910
- Liposomes from Soya Phospholipids as Percutaneous Drug Carriers. *Arzneimittel Forschung Drug Research,* 1990, vol. 40, 1365-1368
- **G. BLUME.** *SÖFW-Journal,* 2000, vol. 11, 14-17
- **G. CEVC.** Transdermal Drug Carriers. *J. Contr. Release,* 1995, vol. 36, 3-16
- **E. TOUITOU.** Ethosomal Carriers. *Drug Dev. Res.,* 2000, vol. 50, 406-415
- **KUMAR ; RAJESHWARRAO.** Nonionic surfactant vesicular systems for effective drug delivery - an overview. *Acta Pharmaceutica Sinica B,* 2011, vol. 1, 208-219